(19) Europäisches Patentamt
European Patent Office
Office européen des brevets

(11) Veröffentlichungsnummer: **0 148 755 B1**

# (12) EUROPÄISCHE PATENTSCHRIFT

(45) Veröffentlichungstag der Patentschrift: **20.11.86**

(51) Int. Cl.⁴: **C 07 C 143/828**

(21) Anmeldenummer: **85100426.7**

(22) Anmeldetag: **18.01.82**

(60) Veröffentlichungsnummer der früheren Anmeldung nach Art. 76 EPÜ: **0056969**

## (54) 2-Phenoxy-phenylsulfonylisocyanat.

(30) Priorität: **26.01.81 JP 8977/81**
**20.06.81 JP 94571/81**

(43) Veröffentlichungstag der Anmeldung: **17.07.85 Patentblatt 85/29**

(45) Bekanntmachung des Hinweises auf die Patenterteilung: **20.11.86 Patentblatt 86/47**

(84) Benannte Vertragsstaaten: **CH DE FR GB IT LI**

(56) Entgegenhaltungen: **US - A - 3 484 466**

(73) Patentinhaber: **NIHON TOKUSHU NOYAKU SEIZO K.K., No.4, 2-chome, Nihonbashi Honcho Chuo-ku, Tokyo 103 (JP)**

(72) Erfinder: **Aya, Masahiro, 2-844, Suzuki-cho, Kodaira-shi Tokyo (JP)**
Erfinder: **Saito, Junichi, 3-7-12, Osawa, Mitaka-shi Tokyo (JP)**
Erfinder: **Yasui, Kazuomi, 7-6-15, Shakujii-dai, Nerima-ku Tokyo (JP)**
Erfinder: **Shiokawa, Kozo, 210-6, Shukugawara Tama-ku, Kawasaki-shi Kanagawa-ken (JP)**
Erfinder: **Morishima, Norihisa, 4-32-5, Owada-cho, Hachioji-shi Tokyo (JP)**
Erfinder: **Goto, Toshio, 5-20-1-304, Seishin, Sagamihara-shi Kanagawa-ken (JP)**

(74) Vertreter: **Schumacher, Günter, Dr. et al, c/o Bayer AG Konzernverwaltung RP Patentabteilung, D-5090 Leverkusen 1 Bayerwerk (DE)**

EP 0 148 755 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft das neue 2-Phenoxy-phenylsulfonylisocyanat der Formel I

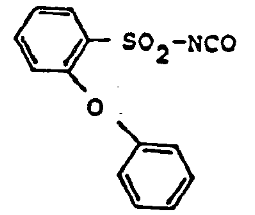

(I)

ein Verfahren zu seiner Herstellung und seine Verwendung als Zwischenprodukt zur Herstellung von herbizid wirksamen 2-Phenoxy-phenylsulfonylharnstoffen.

Es wurde gefunden, dass man das als Zwischenprodukt gewünschte 2-Phenoxy-phenylsulfonylisonat der Formel (I) herstellen kann, indem man 2-Phenoxy-phenylsulfonamid der Formel II

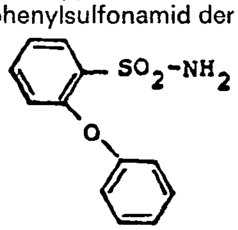

(II)

in Gegenwart eines aliphatischen Isocyanats und gegebenenfalls in Gegenwart eines Verdünnungsmittels mit Phosgen ($COCl_2$) oder Chlorameisensäure-trichlormethylester ($CCl_3O-CO-Cl$) umsetzt.

Verwendet man neben 2-Phenoxy-phenylsulfonamid Phosgen als Ausgangsstoff und n-Butylisocyanat als aliphatisches Isocyanat, so kann der Reaktionsablauf zusammenfassend durch folgendes Formelschema wiedergegeben werden:

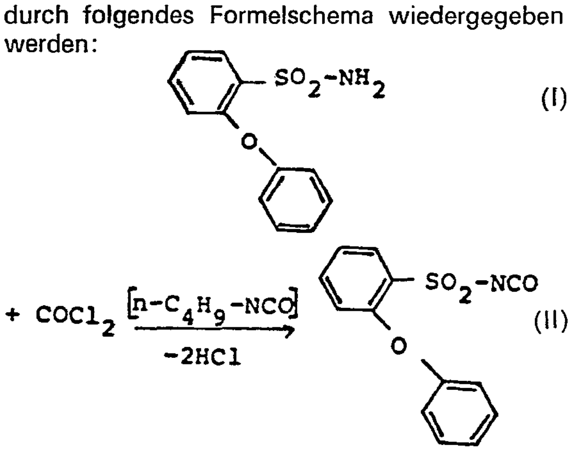


Es ist notwendig, dass diese Umsetzung in Gegenwart eines aliphatischen Isocyanats durchgeführt wird; dieses kann nach Beendigung der Reaktion wiedergewonnen und neu eingesetzt werden. Bevorzugt verwendet man Alkylisocyanate mit 3-8 C-Atomen oder Cycloalkylisocyanate mit 5-8 C-Atomen. Als Beispiele hierfür seien im einzelnen genannt: n-Butylisocyanat, n-Hexylisocyanat und Cyclohexylisocyanat.

Als Verdünnungsmittel für dieses Verfahren kommen alle üblichen inerten Lösungsmittel in Frage, beispielsweise Kohlenwasserstoffe wie Hexan, Cyclohexan, Petrolether, Ligroin, Toluol, Xylol; chlorierte Kohlenwasserstoffe wie z.B. Methylenchlorid, Chloroform, Tetrachlorkohlenstoff, Ethylenchlorid, Trichlorethylen oder Chlorbenzol; Ether wie Diethylether, Methylethylether, Di-isopropylether, Dibutylether, Dioxan, Tetrahydrofuran; Ketone wie z.B. Aceton, Methylethylketon, Methylisopropylketon oder Methylisobutylketon; Nitrile wie z.B. Acetonitril oder Propionitril; Ester wie z.B. Essigsäureethyl oder -amylester; Amide wie z.B. Dimethylformamid oder Dimethylacetamid; Sulfone und Sulfoxide wie z.B. Sulfolan und Dimethylsulfoxid; ferner Basen wie z.B. Pyridin.

Es ist vorteilhaft, die Umsetzung (II) → (I) in Gegenwart eines geeigneten Katalysators durchzuführen. Als geeignete Katalysatoren sind insbesondere tertiäre Amine zu nennen, wie z.B. Triethylamin, Dimethylcyclohexylamin und 1,4-Diazabicyclo(2.2.2)-octan.

Die Reaktionstemperaturen können bei der Umsetzung (II) → (I) in einem weiten Bereich variiert werden. Im allgemeinen arbeitet man bei Temperaturen zwischen −20° C und dem Siedepunkt des Reaktionsgemisches, vorzugsweise zwischen 80 und 150° C. Die Umsetzung wird im allgemeinen bei Normaldruck durchgeführt, sie kann jedoch auch bei erhöhtem oder vermindertem Druck durchgeführt werden.

Bei der Durchführung der Umsetzung (II) → (I) setzt man im allgemeinen auf 1 Mol Phenylsulfonamid (II) 1-2 Mol, vorzugsweise 1,05-1,3 Mol des aliphatischen Isocyanats und 1-4 Mol, vorzugsweise 1-1,5 Mol Phosgen bzw. 0,5-1 Mol, vorzugsweise 0,55-0,65 Mol Chlorameisensäure-trichlormethylester ein. Der Katalysator wird in Mengen von 0,1-5 g, vorzugsweise von 0,8-2,2 g pro Mol (II) eingesetzt.

Die Aufarbeitung und Isolierung des 2-Phenoxy-phenylsulfonylisocyanats (I) erfolgt in üblicher Weise; das Isocyanat (I) kann beispielsweise durch Destillation gereinigt werden.

Das Herstellungsverfahren (II) → (I) wird durch das nachfolgende Synthesebeispiel weiter erläutert:

*Synthesebeispiel*

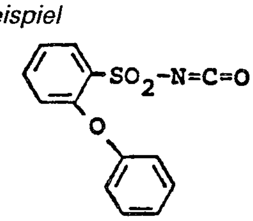

(I)

25,0 g 2-Phenoxy-phenylsulfonamid, 14,0 g n-Hexylisocyanat, eine katalytische Menge 1,4-Diazabicyclo[2.2.2]-octan und 150 ml Xylol wurden unter Rühren eine Stunde unter Rückfluss erhitzt. Dann wurde im Verlauf von 2,5 Stunden eine Lösung von 11,9 g Chlorameisensäure-trichlormethylester in 30 ml Xylol tropfenweise hinzugefügt, wobei die Innentemperatur bei 120-125° C gehalten wurde. Nach der Zugabe wurde die gleiche Temperatur noch einige Zeit aufrechterhalten und danach der Inhalt zur Vervollständigung der Reaktion noch kurze Zeit zum Rückfluss erhitzt. Nach

Beendigung der Reaktion wurde Xylol und n-Hexylisocyanat unter vermindertem Druck abdestilliert, wonach 22,8 g des gewünschten 2-Phenoxy-phenylsulfonylisocyanats erhalten wurden. Siedepunkt: 146-150° C/0,93 mbar (0,7 mmHg).

Das erfindungsgemässe 2-Phenoxy-phenylsulfonylisocyanat (I) kann als Zwischenprodukt zur Herstellung von herbizid wirksamen 2-Phenoxy-phenylsulfonylharnstoffen der Formel III

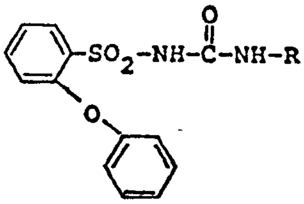

(III)

dienen, in der R für die Gruppe

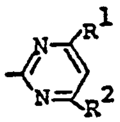

oder

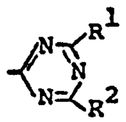

steht, worin $R^1$ und $R^2$ eine Methyl-Gruppe oder eine Methoxy-Gruppe bezeichnen (vgl. Stammanmeldung Nr. 82100319.1/EP-A2-0 056 969).

Man erhält die neuen Wirkstoffe der Formel (III), wenn man das 2-Phenoxy-phenylsulfonylisocyanat (I) mit einer Aminoverbindung der Formel IV

$$NH_2-R \qquad (IV)$$

in der R die vorstehend genannte Bedeutung hat, vorzugsweise in Anwesenheit eines inerten Lösungsmittels oder Verdünnungsmittels und gegebenenfalls in Gegenwart eines Katalysators bei einer Temperatur zwischen −20° C und dem Siedepunkt des Reaktionsgemisches, vorzugsweise zwischen 0° C und 100° C, umsetzt.

Besonders geeignete Aminoverbindungen der Formel (IV) sind die folgenden:

2-Amino-4,6-dimethylpyrimidin,
2-Amino-4-methoxy-6-methylpyrimidin,
2-Amino-5-methoxy-6-methyl-1,3,5-triazin,
2-Amino-4,6-dimethoxy-1,3,5-triazin.

*Anwendungsbeispiele*

a) 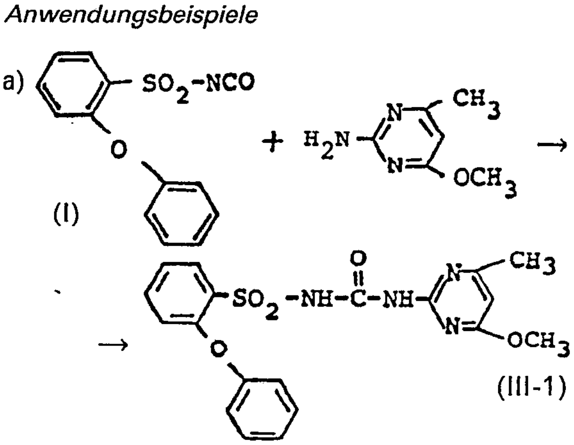

(I)

(III-1)

13,9 g 2-Amino-4-methoxy-6-methylpyrimidin werden in 150 ml trockenem Dichloromethan aufgelöst. Zu dieser Lösung wird im Verlauf einer Stunde tropfenweise eine Lösung von 27,5 g 2-Phenoxy-phenylsulfonylisocyanat (I) in 40 ml Toluol hinzugefügt.

Während der Zugabe wird die Innentemperatur auf Raumtemperatur gehalten. Nach Beendigung der Zugabe wird die Mischung zur Vervollständigung der Reaktion 10 Stunden bei Raumtemperatur gerührt. Nach Beendigung der Reaktion wird die Mischung durch Abdampfen des Dichloromethans unter vermindertem Druck etwa auf die Hälfte ihres Volumens eingeengt, und die dabei gebildeten farblosen Kristalle werden abfiltriert. Die Kristalle werden dann mit einer kleinen Menge Ether gewaschen und getrocknet, wonach 35,2 g (≙ 92,1% d. Th.) des gewünschten Produkts, N-(2-Phenoxy-phenylsulfonyl)-N'-(4-methoxy-6-methylpyrimidin-2-yl)-harnstoff, Schmelzpunkt: 196-200° C, erhalten werden.

In analoger Weise können auch die in der nachfolgenden Tabelle 1 aufgeführten 2-Phenoxyphenylsulfonylharnstoffe der Formel (III) hergestellt werden:

*Tabelle 1*

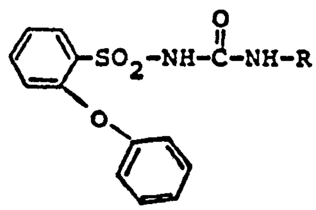

(III)

| Beispiel Nr. | Verbindung Nr. | R | Physik. Konstante Schmp. (° C) |
|---|---|---|---|
| b) | (III-2) | 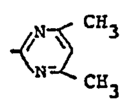 | 178-180 |
| c) | (III-3) | 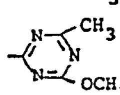 | 185-190 |
| d) | (III-4) | 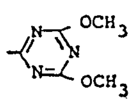 | 160-165 |

Im Vergleich zu bekannten Wirkstoffen mit ähnlicher Struktur und Aktivität (vgl. z.B. die japanische Offenlegungsschrift Sho 52-122384) sind die neuartigen Verbindungen der allgemeinen Formel (III) dadurch gekennzeichnet, dass sie eine wesentlich verbesserte Wirkung zeigen und keine oder nur eine sehr geringe Toxizität gegenüber Warmblütern aufweisen, und aus diesem Grunde sind die neuen Verbindungen sehr wertvoll.

Die hervorragenden Vorteile und bedeutsamen Wirkungen der Wirkstoffe (III) können aus den folgenden Ergebnissen eines Tests ersehen werden, in dem diese Verbindungen gegen verschiedene Unkräuter angewandt wurden.

*Test-Beispiel*

Test zur Behandlung von Stengeln und Blättern sowie des Bodens unter Bewässerungsbedingungen gegen in Wasser wachsende Reis-Unkräuter (Pot test):

*Herstellung der den Wirkstoff enthaltenden Präparate:*

Ein Präparat des speziellen Wirkstoffs der Formel (III) kann durch Vermischen eines Gewichtsteils des Wirkstoffs, 5 Gewichtsteilen Aceton als Träger und 1 Gewichtsteil Emulgator unter Bildung einer Emulsion hergestellt werden. Eine bestimmte Menge dieses Präparats wird dann mit Wasser verdünnt.

*Test-Verfahren:*

Reis-Kulturboden wurde in Wagner-Töpfe von 2 $dm^2$ Fläche gefüllt, und Reis-Setzlinge (Sortenbezeichnung: Kinmaze) im 2-3-Blattstadium (Pflanzenhöhe etwa 10 cm) wurden umgepflanzt, jeweils 2 Setzlinge pro Topf. Dann wurden Samen von Echinochloa crus-galli Beauv. var., Cyperus iria L., Monochoria vaginalis Presl., Scirpus juncoides Roxburgh var. sowie Bruchstücke breitblättriger Unkräuter von Eleocharis acicularis L. und Wurzeln von Cyperus serotinus Rottboell und Sagittaria pygmaea Miq. inokuliert und Feuchtbedingungen aufrechterhalten. Nachdem Echinochloa crus-galli Beauv. var. etwa bis zum 2-Blattstadium (7-9 Tage nach dem Inokulieren) gewachsen war, wurde Wasser bis zu einer Tiefe von etwa 6 cm eingefüllt und eine vorher festgelegte Menge jeder der Verbindungen gemäss der Erfindung in Form einer Emulsion durch Pipettieren dem Wasser zugesetzt. Das Wasser wurde mit einer Geschwindigkeit von 2-3 cm Tiefe pro Tag während zweier Tage nach der Behandlung abgezogen, und danach wurden die Wassertiefe in den Töpfen bei etwa 3 cm gehalten. Vier Wochen nach der chemischen Behandlung wurde die herbizide Wirkung und der Grad der Phytotoxizität auf einer Skala von 0-5 nach dem folgenden Massstab bewertet.

Die Bewertung der Wirkung erfolgt auf der Basis der Unkrautvernichtungsrate im Verhältnis zu derjenigen einer unbehandelten Fläche wie folgt:

5: 95% oder höher (Tod)
4: 80% (einschl.) - 95% (ausschl.)
3: 50% (einschl.) - 80% (ausschl.)
2: 30% (einschl.) - 50% (ausschl.)
1: 10% (einschl.) - 30% (ausschl.)
0: weniger als 10% (keine Wirkung)

Die Bewertung der Phytotoxizität gegenüber Reispflanzen wird aufgrund der Phytotoxizität relativ zu derjenigen auf unbehandelten Flächen vorgenommen und durch folgende Wertzahlen ausgedrückt:

5: 90% oder höher (Ausrottung)
4: 50% (einschl.) - 90% (ausschl.)
3: 30% (einschl.) - 50% (ausschl.)
2: 10% (einschl.) - 30% (ausschl.)
1: weniger als 10%, jedoch nicht 0
0: 0% (keine Phytotoxizität)

Die Ergebnisse sind in Tabelle 2 aufgeführt.

*Tabelle 2*

| Verbindung Nr. | Aufwandmenge des Wirkstoffs kg/ha | Herbizide Wirkung Unkräuter A | B | C | D | E | F | G | H | Phytotoxizität Reis |
|---|---|---|---|---|---|---|---|---|---|---|
| (III-1) | 0,2 | 5 | 5 | 5 | 5 | 5 | 5 | 5 | 5 | 0 |
| (III-2) | 0,2 | 5 | 5 | 5 | 5 | 5 | 5 | 5 | 5 | 0 |
| (III-3) | 0,2 | 5 | 5 | 5 | 5 | 5 | 5 | 5 | 5 | 0 |
| (III-4) | 0,2 | 5 | 5 | 5 | 5 | 5 | 5 | 5 | 5 | 0 |
| Vergleichs-Verbindung (V-1) | 0,2 | 5 | 5 | 5 | 5 | 5 | 5 | 3 | 5 | 4 |

*Anmerkungen*

1. Die Nummern der Verbindungen entsprechen denjenigen, die in den obigen Anwendungsbeispielen und der obigen Tabelle 1 angegeben sind.

2. Die Symbole A, B, C, D, E, F, G und H stehen für die folgenden Unkräuter:
A: Echinochloa crus-galli Beauv. var.,
B: Eleocharis acicularis L.,
C: Cyperus iria L.,
D: Scirpus juncoides Roxburgh var.,
E: Monochoria vaginalis Presl.,
F: Breitblättrige Unkräuter (wie Lindernia Procumbens Philcox, Rotala indica Koehne, Elatine triandra Schk. etc.),
G: Cyperus serotinus Rottboell,
H: Sagittaria pygmaea Miq.

3. Die Vergleichsverbindung (V-1) ist N-(2-Chlorophenylsulfonyl)-N'-(4-methoxy-6-methyl-1,3,5-triazin-2-yl)-harnstoff der Formel (V-1)

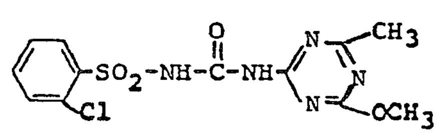

(V-1)

(eine vorbekannte, in der japanischen Offenlegungsschrift Sho 52-122384 beschriebene Verbindung).

## Patentansprüche

1. 2-Phenoxy-phenylsulfonylisocyanat der Formel I

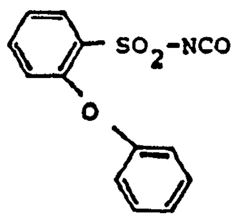

(I)

2. Verfahren zur Herstellung von 2-Phenoxy-phenylsulfonylisocyanat, dadurch gekennzeichnet, dass man 2-Phenoxy-phenylsulfonamid der Formel (II)

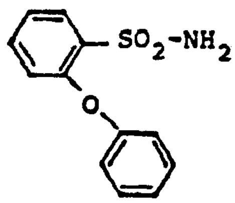

(II)

in Gegenwart eines aliphatischen Isocyanats und gegebenenfalls in Gegenwart eines Verdünnungsmittels mit Phosgen ($COCl_2$) oder Chlorameisensäure-trichlormethylester ($CCl_3O-CO-Cl$) umsetzt.

## Claims

1. 2-Phenoxyphenylsulphonylisocyanate of the formula I

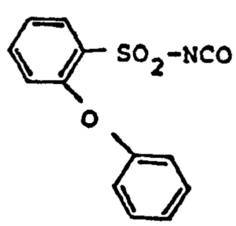

(I)

2. Process for the preparation of 2-phenoxyphenylsulphonylisocyanate, characterised in that 2-phenoxyphenylsulphonamide of the formula (II)

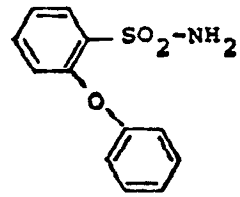

(II)

is reacted with phosgene ($COCl_2$) or chloroformic acid trichloromethyl ester ($CCl_3O-CO-Cl$) in the presence of an aliphatic isocyanate and if appropriate in the presence of a diluent.

## Revendications

1. Le 2-phénoxy-phénylsulfonylisocyanate de formule (I)

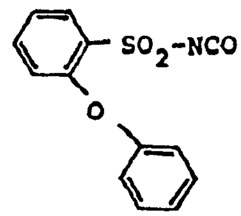

(I)

2. Procédé de préparation du 2-phénoxy-phénylsulfonylisocyanate, caractérisé en ce qu'on fait réagir le 2-phénoxy-phénylsulfonamide de formule (II)

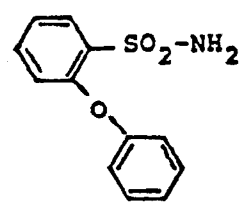

(II)

en présence d'un isocyanate aliphatique et, le cas échéant, en présence d'un diluant, avec le phosgène ($COCl_2$) ou l'ester trichlorométhylique de l'acide chloroformique ($CCl_3O-CO-Cl$).